# EUROPEAN PATENT SPECIFICATION

(11) **EP 1 797 196 B1**
(45) Date of publication and mention of the grant of the patent: **21.08.2013**
(21) Application number: 05789422.2
(22) Date of filing: 06.10.2005
(51) Int. Cl.: C12Q 1/68

(54) **DETECTION OF BREAST CANCER**
NACHWEIS VON BRUSTKREBS
DETECTION DU CANCER DU SEIN

(30) Priority: 06.10.2004 GB 0422211
(43) Date of publication of application: 20.06.2007
(73) Proprietor: Randox Laboratories Ltd., Crumlin, Co. Antrim Northern Ireland, BT29 4QY (GB)
(72) Inventor: CROCKARD, Martin, Andrew, Co. Antrim Northern Ireland BT29 4QY (GB); LAMONT, John, Victor, Co- Antrim Northern Ireland BT29 4QY (GB); FITZGERALD, Stephen, P., Co. Antrim Northern Ireland BT29 4QY (GB)
(74) Representative: Jappy, John William Graham
(86) International application number: PCT/GB2005/003841
(87) International publication number: WO 2006/038010

(56) References cited:
- WO-A-02/055988
- WO-A-03/087840
- WO-A-03/106648
- WO-A-2004/046382
- DATABASE Geneseq [Online] 7 October 2004 (2004-10-07), "Human tumour-associated antigenic target (TAT) cDNA sequence #1146." XP002369393 retrieved from EBI accession no. GSN:ADQ84332 Database accession no. ADQ84332 -& WO 2004/060270 A (GENENTECH, INC; WU, THOMAS, D; ZHOU, YAN) 22 July 2004 (2004-07-22)
- DATABASE EMBL-SVA [Online] 18 March 2002 (2002-03-18), "Homo sapiens mRNA; EST DKFZp686B17117_r1 (from clone DKFZp686B17117)" XP002369394 retrieved from EBI accession no. EM_EST:HSM044457 Database accession no. HSM044457
- PENG LIANG ET AL: "DIFFERENTIAL DISPLAY AND CLONING OF MESSENGER RNAS FROM HUMAN BREAST CANCER VERSUS MAMMARY EPITHELIAL CELLS" CANCER RESEARCH, AMERICAN ASSOCIATION FOR CANCER RESEARCH, BALTIMORE, MD, US, vol. 52, 15 December 1992 (1992-12-15), pages 6966-6968, XP002032010 ISSN: 0008-5472

## Description

### Field of the Invention

This invention relates to the detection of the presence of, or the risk of breast cancer.

### Background of the Invention

There are over 1 million cases of breast cancer per year on a global basis, of which around 0.5 million are in the US, 40,000 are in the UK and nearly 2,000 in Ireland. It is the leading cause of cancer deaths among women (Keen and Davidson, 2003). Although the overall incidence of the disease is increasing within the western world, wider screening and improved treatments have led to a gradual decline in the fatality rate of about 1 % per year since 1991. Inheritance of susceptibility genes, such as BRCA1 and BRCA2, account for only 5% of breast cancer cases and the factors responsible for the other 95% remain obscure (Grover and Martin, 2002). In the absence of a strategy to reduce causative agents of breast cancer, early detection remains the best approach to reducing the mortality rate of this disease. It is widely held that breast cancer initiates as the pre-malignant stage of atypical ductal hyperplasia (ADH), progresses into the pre-invasive stage of ductal carcinoma in situ (DCIS), and culminates in the potentially lethal stage of invasive ductal carcinoma (IDC). This linear model of breast cancer progression has been the rationale for the use of detection methods such as mammography in the hope of diagnosing and treating breast cancer at earlier clinical stages (Ma et ail.,2003).

Patients diagnosed with early breast cancer have greater than a 90% 5 year relative survival rate, as compared to 20% for patients diagnosed with distally metastasised breast cancer. Nonetheless, there is no definitive early-stage screening test for breast cancer, diagnosis currently being made on the results of mammography and fine needle biopsy. Mammography has its limitations, with over 80% of suspicious results being false positives and 10-15% of women with breast cancer providing false negative results. Often the tumour has reached a late stage in development before detection, reducing the chances of survival for the patient and increasing the cost of treatment and management for the healthcare system. More sensitive methods are required to detect small (<2 cm diameter) early stage in-situ carcinomas of the breast, to reduce patient mortality. In addition to early detection, there remain serious problems in classifying the disease as malignant or benign, in the staging of known cancers and in differentiating between tumour types. Finally, there is a need to monitor ongoing treatment effects and to identify patients becoming resistant to particular therapies. Such detection processes are further complicated, as the mammary gland is one of the few organs that undergo striking morphological and functional changes during adult life, particularly during pregnancy, lactation and involution, potentially leading to changes in the molecular signature of the same mammary gland over time.

Diagnosis of disease is often made by the careful examination of the relative levels of a small number of biological markers. Despite recent advances, the contribution of the current biomarkers to patient care and clinical outcome is limited. This is due to their low diagnostic sensitivity and disease specificity. Some molecular biomarkers, however, are being used routinely in disease diagnosis, for example prostate specific antigen in prostate cancer screening, and new candidate markers are being discovered at an increasing rate (Pritzker, 2002). It is becoming accepted that the use of a panel of well-validated biomarkers would enhance the positive predictive value of a test and minimize false positives or false negatives (Srinivas et al., 2002). In addition, there is now growing interest in neural networks, which show the promise of combining weak but independent information from various biomarkers to produce a prognostic/predictive index that is more informative than each biomarker alone (Yousef et al., 2002).

As more molecular information is being collated, diseases such as breast cancer are being sub-divided according to genetic signatures linked to patient outcome, providing valuable information for the clinician. Emerging novel technologies in molecular medicine have already demonstrated their power in discriminating between disease sub-types that are not recognisable by traditional pathological criteria (Sorlie et al., 2001) and in identifying specific genetic events involved in cancer progression (Srinivas ef a/., 2002). Further issues need to be addressed in parallel, relating to the efficacy of biomarkers between genders and races, thus large scale screening of a diverse-population is a necessity.

In addition, the management of breast cancer could be improved by the use of new markers normally expressed only in the breast but found elsewhere in the body, as a result of the disease. Predictors of the activity of the disease would also have valuable utility in the management of the disease, especially those that predict if a ductal carcinoma in situ will develop into invasive ductal carcinoma.

### Summary of the Invention

According to a first aspect of the present invention, there is an *in vitro* method for the detection of the presence of or the risk of breast cancer in a genome sample obtained from a patient, comprising the step of:
(i) detecting in the sample the presence or expression of the gene characterised by the nucleotide sequence identified as SEQ ID No. 10 or its complement, or a polynucleotide of at least 15 consecutive nucleotides that hybridises to the sequence or its complement under stringent hybridising conditions, wherein increased expression of the gene in the sample compared to the expression level in normal tissue indicates the presence of or the risk of breast cancer.

According to a second aspect of the invention, an isolated polynucleotide consists of the sequence identified herein as SEQ ID. No. 10, or its complement, or a fragment thereof that contains at least 15 consecutive nucleotides.

According to a third aspect of the present invention, an isolated peptide consists of the sequence identified herein as SEQ ID No. 12 or a fragment thereof of at least 10 consecutive amino acid residues.

According to a fourth aspect of the present invention, an antibody has an affinity of at least 10⁻⁶M for a peptide as defined above.

According to a fifth aspect of the invention, there is provided the use of a polynucleotide of the present invention, in an *in vitro* diagnostic assay to test for the presence of or the risk of breast cancer in a patient.

### Description of the Invention

The present invention is based on the identification of genes that are expressed in a patient suffering breast cancer. Identification of the individual genes (or their expressed products) in a sample obtained from a patient indicates the presence of or the risk of cancer in the patient.

The invention further relates to reagents such as polypeptide sequences, useful for detecting, diagnosing, monitoring, prognosticating, preventing, imaging, treating or determining a pre-disposition to cancer.

The methods to carry out the diagnosis can involve the synthesis of cDNAfrom the mRNA in a test sample, amplifying as appropriate portions of the cDNA corresponding to the genes or fragments thereof and detecting each product as an indication of the presence of the disease in that tissue, or detecting translation products of the mRNAs comprising gene sequences as an indication of the presence of the disease.

Useful reagents include polypeptides or fragment(s) thereof which may be useful in diagnostic methods such as RT-PCR, PCR or hybridisation assays of mRNA extracted from biopsied tissue, blood or other test samples; or proteins which are the translation products of such mRNAs; or antibodies directed gainst these proteins. These assays also include methods for detecting the gene products (proteins) in light of possible post-translation modifications that can occur in the body, including interactions with molecules such as co-factors, inhibitors, activators and other proteins in the formation of sub-unit complexes.

Diagnosis can be made on the basis of the presence or absence of the gene product or by measuring increased expression of the gene in the patient.

The genes associated with cancer, are characterised by the polynucleotide shown as SEQ ID No. 10. The expressed product of the genes are identified herein by SEQ ID No. 12, respectively. Identification of the genes or their expressed products may be carried out by conventional techniques known for the detection or characterisation of polynucleotides or polypeptides. For example, isolated genetic material from a patient can be probed using short oligonucleotides that hybridise specifically to the target gene. The oligonucleotide probes may be detectably labelled, for example with a fluorophore, so that upon hybridisation with the target gene, the probes can be detected. Alternatively, the gene, or parts thereof, may be amplified using the polymerase chain reaction, with the products being identified, again using labelled oligonucleotides.

Diagnostic assays incorporating any of these genes, proteins or antibodies will include, but not be limited to:
Polymerase chain reaction (PCR)
Reverse transcription PCR
Real-time PCR
In-situ hybridisation
Southern dot blots
Immuno-histochemistry
Ribonuclease protection assay
cDNA array techniques
ELISA

Protein, antigen or antibody arrays on solid supports such as glass or ceramics.

Small interfering RNA functional assays.

All of the above techniques are well known to those in the art.

The present invention is also concerned with isolated polynucleotides that consist of the sequence identified as SEQ ID No. 10 or its complement, or fragments thereof that comprise at least 15 consecutive nucleotides, preferably 30 nucleotides, more preferably at least 50 nucleotides. Polynucleotides that hybridise to a polynucleotide as defined above, are also within the scope of the invention. Hybridisation will usually be carried out under stringent conditions, known to those in the art and are chosen to reduce the possibility of non-complementary hybridisation. Examples of suitable conditions are disclosed in Nucleic Acid Hybridisation. A Practical Approach (B. D. Hames and S. J. Higgins, editors IRL Press, 1985).

The identification of the the TS32 gene (SEQ ID No. 10) also permits therapies to be developed, with each gene being a target for therapeutic molecules. For example, there are now many known molecules that have been developed for gene therapy, to target and prevent the expression of a specific gene. One particular molecule is a small interfering RNA (siRNA), which suppresses the expression of a specific target protein by stimulating the degradation of the target mRNA. Other synthetic oligonucleotides are also known which can bind to a gene of interest (or its regulatory elements) to modify expression. Peptide nucleic acids (PNAs) in association with DNA (PNA-DNA chimeras) have also been shown to exhibit strong decoy activity, to alter the expression of the gene of interest.

The present invention also includes antibodies raised against a peptide expressed by any of the genes identified in the invention. The antibodies will usually have an affinity for the peptide of at least 10⁻⁶ M, more preferably, 10⁻⁹ M and most preferably at least 10⁻¹¹ M. The antibody may be of any suitable type, including monoclonal or polyclonal. Assay kits for determining the presence of the peptide antigen in a test sample are also included. In one embodiment, the assay kit comprises a container with an antibody, which specifically binds to the antigen, wherein the antigen comprises at least one epitope encoded by the TS32 gene. These kits can further comprise containers with useful tools for collecting test samples, such as blood, saliva, urine and stool. Such tools include lancets and absorbent paper or cloth for collecting and stabilising blood, swabs for collecting and stabilising saliva, cups for collecting and stabilising urine and stool samples. The antibody can be attached to a solid phase, such as glass or a ceramic surface.

Detection of antibodies that specifically bind to any of the antigens in a test sample suspected of containing these antibodies may also be carried out. This detection method comprises contacting the test sample with a polypeptide, which contains at least one epitope of the gene. Contacting is performed for a time and under conditions sufficient to allow antigen/antibody complexes to form. The method further entails detecting complexes, which contain any of the polypeptides. The polypeptide complex can be produced recombinantly or synthetically or be purified from natural sources.

Antibodies, or fragments thereof, against any of the antigens (peptides) can be used for the detection of image localisation of the antigen in a patient for the purpose of detecting or diagnosing the disease or condition. Such antibodies can be monoclonal or polyclonal, or made by molecular biology techniques and can be labelled with a variety of detectable agents, including, but not limited to radioisotopes.

Antibodies or fragments thereof, whether monoclonal or polyclonal or made by molecular biology techniques, can be used as therapeutics for the disease characterised by the expression of any of the genes of the invention. The antibody may be used without derivatisation, or it may be derivatised with a cytotoxic agent such as radioisotope, enzyme, toxin, drug, pro-drug or the like.

The term "antibody "refers broadly to any immunologic binding agent such as IgG, IgM, IgA, IgD and IgE. Antibody is also used to refer to any antibody-like molecule that has an antigen-binding region and includes antibody fragments such as single domain antibodies (DABS), Fv, scFv, aptamers, etc. The techniques for preparing and using various antibody-based constructs and fragments are well known in the art. Means for preparing and characterising antibodies are also well known in the art.

If desired, the cancer screening methods of the present invention may be readily combined with other methods in order to provide an even more reliable indication of diagnosis or prognosis, thus providing a multi-marker test.

The following examples illustrate the invention with reference to the accompanying drawings.

### Examples

A number of differentially expressed gene fragments were isolated from cDNA populations derived from matched clinical samples of breast cancer patients, using non-isotopic differential display (DDRT-PCR). One of these fragments TS32, showed a significant increase in expression in breast tumour tissue samples from a number of donors, in comparison to their co-excised normal tissue counterparts. The expression profile of these novel molecular markers, their full length and corresponding presumed protein sequences are detailed herein.

### Materials and methods

Differential gene expression was investigated between matched pairs of normal mammary and tumour tissue from the same donor. Tissue samples were obtained, with full ethical approval and informed patient consent, from Medical Solutions pic, Nottingham, UK. Following the surgical removal of a tumour, one sample of the tumour tissue was collected, as was a sample from the adjacent, co-excised normal tissue. Messenger RNA was extracted and cDNA subsequently synthesised, using Dynal dT18-tagged Dynabeads and Superscript II reverse transcription protocols, respectively. Differential display reverse transcription PCR (DDRT-PCR) was employed to observe differences between the gene expression profiles of these matched samples and individual gene transcripts showing up- or down-regulation were isolated and investigated further.

First described by Liang & Pardee (1992) differential display reverse transcription PCR (DDRT-PCR) uses mRNA from two or more biological samples as templates for representative cDNA synthesis by reverse transcription, with one of 3 possible anchor primers. Each of the 3 subpopulations was PCR amplified using its respective anchor primer coupled with one of 80 arbitrary 13-mer primers. This number of primer combinations has been estimated to facilitate the representation of 96% of expressed genes in an mRNA population (Sturtevant, 2000). This population sub-division results in the reduction of the estimated 12,000-15,000 mRNAs expressed in eukaryotic cells to 100-150 transcripts on completion of second strand cDNA synthesis for each primer set. This facilitates the parallel electrophoretic separation and accurate visualization of matched primer sets on a polyacrylamide gel, leading to the identification of gene fragments expressed in one tissue sample but not the other.

Excision and re-amplification of fragments of interest was followed by removal of false positives through reverse Southern dot blotting. This entailed the spotting of each re-amplified fragment onto duplicate nylon membranes (Hybond N+, Amersham Pharmacia Biotech) and hybridising these with either the tumour or normal tissue cDNA population of the donor from which the fragments were derived. Those fragments confirmed as differentially expressed were then cloned and sequenced, followed by web-based database interrogation to determine if each gene was novel and to find its chromosomal location. Fragments not matching known genes were regarded as potentially representing novel markers for the breast cancer from which they were derived. Further screening of each transcript was performed by either semi-quantitative RT-PCR or real-time PCR, using a suite of matched cDNA populations from a number of breast tumour donors. In all cases, β-actin was used as a constitutive reference gene, for calibrating the cDNA templates and as an internal positive control during PCR. Expression of each putative novel marker gene was performed by PCR using gene-specific primer sets on the calibrated matched templates. Potential full-length transcripts of the novel gene fragments, including the open reading frame(that piece of the gene that encodes the protein) were then synthesized using 5<1> RACE (rapid amplification of cDNA ends), which incorporates gene-specific extension and amplification, verifiable by sequencing. Alternatively, homologous known sequences to the putative markers were exploited, with primers being designed for their corresponding open reading frames, followed by sequence verification of the amplified products.

The tissue specific expression profile of each molecular marker was tested using gene specific primers against cDNA populations derived from a comprehensive panel of 22 human tissue types. These are as follows:

| | |
|---|---|
| Adrenal gland | pooled from 62 donors |
| Bone marrow | pooled from 7 donors |
| Brain, cerebellum | pooled from 24 donors |
| Brain, whole | pooled from one donor |
| Colon* | pooled from one donor |
| Foetal brain | pooled from 59 donors |
| Foetal liver | pooled from 63 donors |
| Heart | pooled from one donor |
| Kidney | pooled from one donor |
| Liver | pooled from one donor |
| Lung | pooled from one donor |
| Placenta | pooled from 7 donors |
| Prostate | pooled from 47 donors |
| Salivary gland | pooled from 24 donors |
| Skeletal muscle | pooled from 2 donors |
| Small intestine* | pooled from one donor |
| Spleen | pooled from 14 donors |
| Testis | pooled from 19 donors |
| Thymus | pooled from 9 donors |
| Thyroid gland | pooled from 65 donors |
| Trachea | pooled from 1 donor |
| Uterus | pooled from 10 donors |

The majority of these samples were part of the Human Total RNA panel II (Clontech), but two RNA samples, marked with asterisks, were obtained separately from Clontech. In addition, assays were performed on a range of ethically approved human tumour samples, obtained through Medical Solutions pic, Nottingham, UK. DNA representative of tumours from ovary, testis, stomach, liver, lung, bladder, colon and pancreas was tested against both [beta]- actin and the putative markers, by real-time and conventional PCR.

In conjunction with novel marker expression analysis, each matched pair of breast tissues was subjected to molecular signature analysis. This entailed using of a suite of primers specific to a number of pre-published breast cancer molecular markers in semi-quantitative RT-PCR against each tissue cDNA. The relationship between each molecular marker was determined, tabulated for each sample and used as a reference, against which the novel markers could be compared. This is with the aim of sub-classifying the tumour types and enabling the association of novel markers against such sub-types, increasing the power of the diagnostic marker considerably.

### TS32

Using differential display, a gene fragment, designated TS32, derived from cDNA populations of matched tissue from a breast cancer donor was observed to have significant up-regulation in the tumour cDNA population in comparison to the corresponding normal tissue cDNA. This 232-nucleotide product; 221 nucleotides without the poly A tail (SEQ ID No 10) was confirmed as differentially expressed by reverse Southern dot blots. Sequence analysis followed by database interrogation determined that TS32 was not homologous to known genes or proteins in the EMBL and SWISSPROT databases, respectively, so was regarded as potentially novel. It was, however, 100% homologous, to a predicted gene (rorchee), on chromosome 11 p11, predicted by Acembly Gene Predictions, sourced through a BLAST search of the human genome. The predicted gene comprises 621 nucleotides (SEQ ID No 11) and contains a presumed ORF of 56 amino acids (SEQ ID No 12).

The TS32 fragment was screened using cDNA populations derived from a number of matched breast tumour tissues donated by other cancer patients. Of the donor samples screened, 14 out of 19 exhibited notable increases in expression, as shown in the table below, confirming TS32 to be a putative molecular marker for the presence of breast tumour.

| | | |
|---|---|---|
| TS32 Increased in tumour | 14 | 73.7% |
| TS32 Increased in normal | 3 | 15.7% |
| TS32 No discernable difference | 1 | 5.3% |
| TS32 No expression evident | 1 | 5.3% |
| Totals | 19 | 100% |

To compare the expression of the predicted gene homologue against the original TS32 fragment, ORF primers were designed for rorchee and used against the matched breast cancer panel; the expression profile of the product derived from the ORF primer set against this tissue panel was found to be the same, so the rorchee gene is considered to be the full-length equivalent of TS32. This molecular marker did not show increased expression in all tumour samples from the matched sets, so may be a useful tool for the sub- classification of breast cancer, either in isolation or as part of an array of marker genes.

TS32 was further tested using cDNA populations derived from a panel of 22 human tissue types by real-time PCR analysis. Of those cDNA populations tested, TS32 was detected in most tissues at low levels (data not shown), indicating that the marker is not tissue specific. In addition, assays were performed on a range of ethically approved human tumour samples, obtained through Medical Solutions, to ascertain whether the marker was breast tumour specific or a less specific tumour marker. TS32 was again present in most tumours at low levels (data not shown), so cannot be regarded as a specific marker for breast cancer. This may therefore have utility as a general indicator for the presence of a tumour, through elevated expression, rather than simply presence or absence.

On the basis of the present results, TS32 can be considered a very strong indicator of cancer in the context of breast specific assays, using biopsy samples, for example. In addition, its lack of uniformity among the matched samples tested may indicate a role in the sub-division of breast tumour types or stages. Higher volume screening is underway to ascertain whether this promising marker can be associated with a particular sub-group of breast cancer or whether it can be used as a marker for other cancer types in addition to breast cancer.

### References

1. DeRisi, J. L., lyer, V. R. and Brown, P. 0. 1997. Exploring the metabolic and genetic control of gene expression on a genomic scale. Science. 278: 680-686.
2. Grover, P. L. and Martin, F. L. 2002. The initiation of breast and prostate cancer. Carcinogenesis. 23 (7): 1095-1102.
3. Hames, B. D. and Higgins, S. J., (Editors). 1985. Nucleic Acid Hybridisation. A Practical Approach. IRL Press.
4. Keen, J. C. and Davidson, N. E. 2003. The biology of breast carcinoma. Cancer. 97 (3-Supplement): 825-833.
5. Liang, P. and Pardee, A. B. 1992. Differential display of eukaryotic messenger RNA by means of the polymerase reaction. Science. 257: 967-971.
6. Ma, Xiao-Jun., Ranelle Salunga, J. Todd Tuggle, Justin Gaudet, Edward Enright, Philip McQuary, Terry Payette, Maria Pistone, Kimberlystecker, Brian M. Zhang, Yi-Xiong Zhou, Heike Varnholt, Barbara Smith, Michelle Gadd, Erica Chatfield, Jessica Kessler, Thomas M. Baer, Mark G. Erlander, and Dennis C. Sgroi. 2003. Gene expression profiles of human breast cancer progression. Proc Natl Acad Sci U S A. 100 (10): 5974-5979.
7. Pritzker, K. P. 2002 Cancer biomarkers: easier said than done. Clin. Chem. 2002 Aug; 48(8): 1147-50.
8. Salodof MacNeil. 2001. From genes to proteins: The FLEXgene consortium. HMS Beagle. 112: on-line journal.
9. Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO,
10. Botstein D, Eystein Lonning P, Borresen-Dale AL. 2001. Gene expression patterns of breast carcinomas distinguish tumor subclasses with clinical implications. Proc Natl Acad Sci USA. Sep 11 ;98(19): 10869-74.
11. Srinivas PR, Verma M1 Zhao Y, Srivastava S. 2002. Proteomics for cancer biomarker discovery. Clin Chem. Aug;48(8):1160-9.
12. Strausberg R.L, Feingold EA, Grouse LH., Derge J.G., Klausner R.D. 2002. Generation and initial analysis of more than 15,000 full-length human and mouse cDNA sequences. Proc. Natl. Acad. Sci. U.S.A. 99(26): 16899-16903.
13. Sturtevant, J. Applications of differential-display reverse transcription-PCR to molecular pathogenesis and medical mycology. Clin Microbiol Rev. 2000 Jul; 13(3):408-27.
14. Yousef etal., 2002 Yousef GM, Scorilas A, Kyriakopoulou LG, Rendl L, Diamandis M, Ponzone R, Biglia N, Giai M, Roagna R, Sismondi P, Diamandis EP. Human kallikrein gene 5 (KLK5) expression by quantitative PCR: an independent indicator of poor prognosis in breast cancer. Clin Chem. 2002 Aug;48(8): 1241 -50.
15. Zong, Q., Schummer, M., Hood, L. and Morris, D. R. 1999. Messenger RNA translation state: the second dimension of high-throughput expression screening. Proc. Natl. Acad. Sci. 96: 10632-10636.

## Claims

1. An *in vitro* method for the detection of the presence of or the risk of breast cancer in a genome sample obtained from a patient, comprising the step of:
(i) detecting in the sample the presence or expression of the gene **characterised by** the nucleotide sequence identified as SEQ ID No. 10 or its complement, or a fragment thereof that contains at least 15 consecutive nucleotides,
wherein increased expression of the gene in the sample compared to the expression level in normal tissue indicates the presence of or the risk of breast cancer.

2. A method according to claim 1, wherein the sample is obtained from breast tissue.

3. A method according to claim 1 or claim 2, wherein detection is carried out by amplifying the gene using the polymerase enzyme.

4. An isolated polynucleotide consisting of the sequence identified herein as SEQ ID. No. 10, or its complement, or a fragment thereof that contains at least 15 consecutive nucleotides.

5. Use of a polynucleotide according to claim 4, in an *in vitro* diagnostic assay to test for the presence of or the risk of breast cancer in a patient.

6. A peptide consisting of the sequence identified herein as SEQ ID No. 12 or a fragment thereof of at least 10 consecutive amino acid residues.

7. An antibody having affinity of at least 10⁻⁶M for the peptide of claim 6.

## Patentansprüche

1. Ein In-vitro-Verfahren zum Nachweis des Vorliegens oder Risikos von Brustkrebs in einer von einem Patienten gewonnenen Genomprobe, das den folgenden Schritt umfasst:
(i) Nachweisen des Vorliegens oder der Expression des Gens in der Probe, das durch die Nukleotidsequenz gekennzeichnet ist, die als SEQ ID Nr. 10 oder ihrem Komplement oder einem Fragment davon, das mindestens 15 aufeinanderfolgende Nucleotide enthält, wobei die erhöhte Expression des Gens in der Probe im Vergleich zu dem Expressionsniveau in normalem Gewebe auf das Vorliegen oder Risiko von Brustkrebs hinweist.

2. Ein Verfahren gemäß Anspruch 1, wobei die Probe aus Brustgewebe gewonnen wird.

3. Ein Verfahren gemäß Anspruch 1 oder Anspruch 2, wobei der Nachweis mittels Amplifizierung des Gens unter Verwendung des Polymeraseenzyms durchgeführt wird.

4. Ein isoliertes Polynucleotid, bestehend aus der Sequenz, die hierin als SEQ ID. Nr. 10 oder ihrem Komplement identifiziert ist, oder einem Fragment davon, das mindestens 15 aufeinanderfolgende Nucleotide enthält.

5. Verwendung eines Polynucleotids gemäß Anspruch 4 in einem diagnostischen In-vitro-Assay zum Testen auf das Vorliegen oder Risiko von Brustkrebs bei einem Patienten.

6. Ein Peptid, bestehend aus der Sequenz, die hierin als SEQ ID. Nr. 12 identifiziert ist, oder einem Fragment davon mit mindestens 10 aufeinanderfolgenden Aminosäureresten.

7. Ein Antikörper mit einer Affinität von mindestens 10⁻⁶ M für das Peptid nach Anspruch 6.

## Revendications

1. Procédé *in vitro* de détection de la présence ou du risque du cancer du sein dans un échantillon génomique obtenu d'un patient, comprenant l'étape de
(i) détection dans l'échantillon de la présence ou de l'expression du gène **caractérisé par** la séquence nucléotidique identifiée comme étant SEQ ID No. 10 ou son complément, ou un fragment de celle-ci qui contient au moins 15 nucléotides consécutifs, dans lequel l'expression accrue du gène dans l'échantillon comparée au niveau d'expression dans le tissu normal indique la présence ou le risque du cancer du sein.

2. Procédé selon la revendication 1, dans lequel l'échantillon est obtenu du tissu mammaire.

3. Procédé selon la revendication 1 ou la revendication 2, dans lequel la détection est réalisée par l'amplification du gène en utilisant une enzyme polymérase.

4. Polynucléotide isolé constitué de la séquence identifiée dans la présente invention comme étant SEQ ID. No. 10, ou son complément, ou un fragment de celle-ci qui contient au moins 15 nucléotides consécutifs.

5. Utilisation d'un polynucléotide selon la revendication 4, dans un dosage diagnostique *in vitro* pour tester pour la présence ou le risque du cancer du sein chez un patient.

6. Peptide constitué de la séquence identifiée selon l'invention comme étant SEQ ID No. 12 ou un fragment de celle-ci d'au moins 10 résidus d'acides aminés consécutifs.

7. Anticorps ayant une affinité d'au moins 10⁻⁶ M pour le peptide selon la revendication 6.


### SEQUENCE LISTING

<110> Randox Laboratories Limited
<120> Detection of Breast Cancer
<130> JWJ01097WO
<150> GB0422211.3
   <151> 2004-10-06
<160> 15
<170> PatentIn version 3.1
<210> 1
   <211> 174
   <212> DNA
   <213> Homo sapiens
<400> 1
   ccagggttct tgtgaagatc aagtgataat gtaggaagca tgtacaaatg tcacattctg     60
   ccgtcacgta atggtcctca cagcttgagg tagcatttag catgtgtcat gatttagtac    120
   aagggttggc aaactgttgc tcttggatta agtctggctc attgcctgtt tttc          174
<210> 2
   <211> 374
   <212> DNA
   <213> Homo sapiens
<400> 2
   caggtccttg gatttctttc tgtgaaaatg aaagcatagg taggaatttc ccatggaaca     60
   gctagcagag gagaaatatt aaaagtcagg agactcatgc tatagttttc atacttcatt    120
   acaacaatgt tgtttaggac aagtgagtta acctgttagc ttcctctata taaaatggaa    180
   agtcattaaa aacctacata gcagggttct tgtgaagatc aagtgataat gtaggaagca    240
   tgtacaaatg tcacattctg ccgtcacgta atggtcctca cagcttgagg tagcatttag    300
   catgtgtcat gatttagtac aagggttggc aaactgttgc tcttggatta agtctggctc    360
   attgcctgtt tttc                                                      374
<210> 3
   <211> 125
   <212> PRT
   <213> Homo sapiens
<220>
   <221> MISC_FEATURE
   <222> (13)..(13)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (35)..(35)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (53)..(53)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (63)..(63)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (67)..(67)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (78)..(78)
   <223> X represents a stop codon encoded by SEQ ID NO:2. (SEQ ID NO:3 i
   ' s the +1 potential amino acid reading frame of SEQ ID NO:2, see p age 12).
<220>
   <221> MISC_FEATURE
   <222> (96)..(96)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (100)..(100)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (104)..(104)
   <223> X represents a stop codon encoded by SEQ ID NO:2. (SEQ ID NO:3 i s the +1 potential amino acid reading frame of SEQ ID NO:2, see p age 12).
<220>
   <221> MISC_FEATURE
   <222> (117)..(117)
   <223> X represents a stop codon encoded by SEQ ID NO:2. (SEQ ID NO:3 i s the +1 potential amino acid reading frame of SEQ ID NO:2, see p age 12).
<400> 3
   Gln Val Leu Gly Phe Leu Ser Val Lys Met Lys Ala Xaa Val Gly Ile
   1               5                   10                  15
   Ser His Gly Thr Ala Ser Arg Gly Glu Ile Leu Lys Val Arg Arg Leu
               20                  25                  30
   Met Leu Xaa Phe Ser Tyr Phe Ile Thr Thr Met Leu Phe Arg Thr Ser
           35                  40                  45
   Glu Leu Thr Cys Xaa Leu Pro Leu Tyr Lys Met Glu Ser His Xaa Lys
       50                  55                  60
   Pro Thr Xaa Gln Gly Ser Cys Glu Asp Gln Val Ile Met Xaa Glu Ala
   65                  70                  75                  80
   Cys Thr Asn Val Thr Phe Cys Arg His Val Met Val Leu Thr Ala Xaa
                   85                  90                  95
   Gly Ser Ile Xaa His Val Ser Xaa Phe Ser Thr Arg Val Gly Lys Leu
               100                 105                 110
   Leu Leu Leu Asp Xaa Val Trp Leu Ile Ala Cys Phe Ser
           115                 120                 125
<210> 4
   <211> 125
   <212> PRT
   <213> Homo sapiens
<220>
   <221> MISC_FEATURE
   <222> (8)..(8)
   <223> X represents a stop codon encoded by SEQ ID NO:2. (SEQ ID NO:4 i s the +2 potential amino-acid reading frame of SEQ ID NO:2, see p age 12) .
<220>
   <221> MISC_FEATURE
   <222> (10)..(10)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (14)..(14)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (27)..(27)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (50)..(50)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (75)..(75)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (76)..(76)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (90)..(90)
   <223> X represents a stop codon encoded by SEQ ID NO:2. (SEQ ID NO:4 i s the +2 potential amino acid reading frame of SEQ ID NO:2, see p age 12).
<220>
   <221> MISC_FEATURE
   <222> (125)..(125)
   <223> X represents a stop codon encoded by SEQ ID NO:2. (SEQ ID NO:4 i s the +2 potential amino acid reading frame of SEQ ID NO:2, see p age 12).
<400> 4
   Arg Ser Leu Asp Phe Phe Leu Xaa Lys Xaa Lys His Arg Xaa Glu Phe
   1               5                   10                  15
   Pro Met Glu Gln Leu Ala Glu Glu Lys Tyr Xaa Lys Ser Gly Asp Ser
               20                  25                  30
   Cys Tyr Ser Phe His Thr Ser Leu Gln Gln Cys Cys Leu Gly Gln Val
           35                  40                  45
   Ser Xaa Pro Val Ser Phe Leu Tyr Ile Lys Trp Lys Val Ile Lys Asn
       50                  55                  60
   Leu His Ser Arg Val Leu Val Lys Ile Lys Xaa Xaa Cys Arg Lys His
   65                  70                  75                  80
   Val Gln Met Ser His Ser Ala Val Thr Xaa Trp Ser Ser Gln Leu Glu
                   85                  90                  95
   Val Ala Phe Ser Met Cys His Asp Leu Val Gln Gly Leu Ala Asn Cys
               100                 105                 110
   Cys Ser Trp Ile Lys Ser Gly Ser Leu Pro Val Phe Xaa
           115                 120                 125
<210> 5
   <211> 124
   <212> PRT
   <213> Homo sapiens
<220>
   <221> MISC_FEATURE
   <222> (21)..(21)
   <223> X represents a stop codon encoded by SEQ ID NO:2. (SEQ ID NO:5 i s the +3 potential amino acid reading frame of SEQ ID NO:2, see p age 12).
<220>
   <221> MISC_FEATURE
   <222> (45)..(45)
   <223> X represents a stop codon encoded by SEQ ID NO:2. (SEQ ID NO:5 i s the +3 potential amino acid reading frame of SEQ ID NO:2, see p age 12).
<220>
   <221> MISC_FEATURE
   <222> (48)..(48)
   <223> X represents a stop codon encoded by SEQ ID NO:2. (SEQ ID NO:5 i s the +3 potential amino acid reading frame of SEQ ID NO:2, see p age 12)
<220>
   <221> MISC_FEATURE
   <222> (57)..(57)
   <223> X represents a stop codon encoded by SEQ ID NO:2. (SEQ ID NO:5 i s the +3 potential amino acid reading frame of SEQ ID NO:2, see p age 12).
<220>
   <221> MISC_FEATURE
   <222> (71)..(71)
   <223> X represents a stop codon encoded by SEQ ID NO:2. (SEQ ID NO:5 i s the +3 potential amino acid reading frame of SEQ ID NO:2, see p age 12).
<220>
   <221> MISC_FEATURE
   <222> (97)..(97)
   <223> X represents a stop codon encoded by SEQ ID NO:2. (SEQ ID NO:5 i s the +3 potential amino acid reading frame of SEQ ID NO:2, see p age 12).
<220>
   <221> MISC_FEATURE
   <222> (105)..(105)
   <223> X represents a stop codon encoded by SEQ ID NO:2. (SEQ ID NO:5 i s the +3 potential amino acid reading frame of SEQ ID NO:2, see p age 12).
<400> 5
   Gly Pro Trp Ile Ser Phe Cys Glu Asn Glu Ser Ile Gly Arg Asn Phe
   1               5                   10                  15
   Pro Trp Asn Ser Xaa Gln Arg Arg Asn Ile Lys Ser Gln Glu Thr His
               20                  25                  30
   Ala Ile Val Phe Ile Leu His Tyr Asn Asn Val Val Xaa Asp Lys Xaa
           35                  40                  45
   Val Asn Leu Leu Ala Ser Ser Ile Xaa Asn Gly Lys Ser Leu Lys Thr
       50                  55                  60
   Tyr Ile Ala Gly Phe Leu Xaa Arg Ser Ser Asp Asn Val Gly Ser Met
   65                  70                  75                  80
   Tyr Lys Cys His Ile Leu Pro Ser Arg Asn Gly Pro His Ser Leu Arg
                   85                  90                  95
   Xaa His Leu Ala Cys Val Met Ile Xaa Tyr Lys Gly Trp Gln Thr Val
               100                 105                 110
   Ala Leu Gly Leu Ser Leu Ala His Cys Leu Phe Phe
           115                 120
<210> 6
   <211> 124
   <212> PRT
   <213> Homo sapiens
<220>
   <221> MISC_FEATURE
   <222> (22)..(22)
   <223> X represents a stop codon encoded by SEQ ID NO:2. (SEQ ID NO:6 i s the -1 potential amino acid reading frame of SEQ ID NO:2, see p age 12).
<220>
   <221> MISC_FEATURE
   <222> (69)..(69)
   <223> X represents a stop codon encoded by SEQ ID NO:2. (SEQ ID NO:6 i s the -1 potential amino acid reading frame of SEQ ID NO:2, see p age 12).
<220>
   <221> MISC_FEATURE
   <222> (75)..(75)
   <223> X represents a stop codon encoded by SEQ ID NO:2. (SEQ ID NO:6 i s the -1 potential amino acid reading frame of SEQ ID NO:2, see p age 12).
<220>
   <221> MISC_FEATURE
   <222> (88)..(88)
   <223> X represents a stop codon encoded by SEQ ID NO:2. (SEQ ID NO:6 i s the -1 potential amino acid reading frame of SEQ ID NO:2, see p age 12).
<220>
   <221> MISC_FEATURE
   <222> (91)..(91)
   <223> X represents a stop codon encoded by SEQ ID NO:2. (SEQ ID NO:6 i s the -1 potential amino acid reading frame of SEQ ID NO:2, see p age 12).
<220>
   <221> MISC_FEATURE
   <222> (98)..(98)
   <223> X represents a stop codon encoded by SEQ ID NO:2. (SEQ ID NO:6 i s the -1 potential amino acid reading frame of SEQ ID NO:2,. see p age 12).
<400> 6
   Lys Thr Gly Asn Glu Pro Asp Leu Ile Gln Glu Gln Gln Phe Ala Asn
   1               5                   10                  15
   Pro Cys Thr Lys Ser Xaa His Met Leu Asn Ala Thr Ser Ser Cys Glu
               20                  25                  30
   Asp His Tyr Val Thr Ala Glu Cys Asp Ile Cys Thr Cys Phe Leu His
           35                  40                  45
   Tyr His Leu Ile Phe Thr Arg Thr Leu Leu Cys Arg Phe Leu Met Thr
       50                  55                  60
   Phe His Phe Ile Xaa Arg Lys Leu Thr Gly Xaa Leu Thr Cys Pro Lys
   65                  70                  75                  80
   Gln His Cys Cys Asn Glu Val Xaa Lys Leu Xaa His Glu Ser Pro Asp
                   85                  90                  95
   Phe Xaa Tyr Phe Ser Ser Ala Ser Cys Ser Met Gly Asn Ser Tyr Leu
               100                 105                 110
   Cys Phe His Phe His Arg Lys Lys Ser Lys Asp Leu
           115                 120
<210> 7
   <211> 125
   <212> PRT
   <213> Homo sapiens
<220>
   <221> MISC_FEATURE
   <222> (5)..(5)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (20)..(20)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (32)..(32)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (41)..(41)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (60)..(60)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (80)..(80)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (85)..(85)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (86)..(86)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (93)..(93)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (96)..(96)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (104)..(104)
   <223> X represents a stop codon encoded by SEQ ID NO:2. (SEQ ID NO:7 i s the -2 potential amino acid reading frame of SEQ ID NO:2, see p age 12).
<220>
   <221> MISC_FEATURE
   <222> (125)..(125)
   <223> X represents a stop codon encoded by SEQ ID NO:2. (SEQ ID NO:7 i s the -2 potential amino acid reading frame of SEQ ID NO:2, see p age 12).
<400> 7
   Lys Asn Arg Gln Xaa Ala Arg Leu Asn Pro Arg Ala Thr Val Cys Gln
   1               5                   10                  15
   Pro Leu Tyr Xaa Ile Met Thr His Ala Lys Cys Tyr Leu Lys Leu Xaa
               20                  25                  30
   Gly Pro Leu Arg Asp Gly Arg Met Xaa His Leu Tyr Met Leu Pro Thr
           35                  40                  45
   Leu Ser Leu Asp Leu His Lys Asn Pro Ala Met Xaa Val Phe Asn Asp
       50                  55                  60
   Phe Pro Phe Tyr Ile Glu Glu Ala Asn Arg Leu Thr His Leu Ser Xaa
   65                  70                  75                  80
   Thr Thr Leu Leu Xaa Xaa Ser Met Lys Thr Ile Ala Xaa Val Ser Xaa
                   85                  90                  95
   Leu Leu Ile Phe Leu Leu Cys Xaa Leu Phe His Gly Lys Phe Leu Pro
               100                 105                 110
   Met Leu Ser Phe Ser Gln Lys Glu Ile Gln Gly Pro Xaa
           115                 120                 125
<210> 8
   <211> 125
   <212> PRT
   <213> Homo sapiens
<220>
   <221> MISC_FEATURE
   <222> (9)..(9)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (26)..(26)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (37)..(37)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (52)..(52)
   <223> X represents a stop codon encoded by SEQ ID NO:2. (SEQ ID NO:8 i s the -3 potential amino acid reading frame of SEQ ID NO:2, see p - age 12).
<220>
   <221> MISC_FEATURE
   <222> (63)..(63)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (64)..(64)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (73)..(73)
   <223> X represents a stop codon encoded by SEQ ID NO:2. (SEQ ID NO:8 i s the -3 potential amino acid reading frame of SEQ ID NO:2, see p age 12).
<220>
   <221> MISC_FEATURE
   <222> (125)..(125)
   <223> X represents a stop codon encoded by SEQ ID NO:2. (SEQ ID NO:8 i s the -3 potential amino acid reading frame of SEQ ID NO:2, see p age 12).
<400> 8
   Glu Lys Gln Ala Met Ser Gln Thr Xaa Ser Lys Ser Asn Ser Leu Pro
   1               5                   10                  15
   Thr Leu Val Leu Asn His Asp Thr Cys Xaa Met Leu Pro Gln Ala Val
               20                  25                  30
   Arg Thr Ile Thr Xaa Arg Gln Asn Val Thr Phe Val His Ala Ser Tyr
           35                  40                  45
   Ile Ile Thr Xaa Ser Ser Gln Glu Pro Cys Tyr Val Gly Phe Xaa Xaa
       50                  55                  60
   Leu Ser Ile Leu Tyr Arg Gly Ser Xaa Gln Val Asn Ser Leu Val Leu
   65                  70                  75                  80
   Asn Asn Ile Val Val Met Lys Tyr Glu Asn Tyr Ser Met Ser Leu Leu
                   85                  90                  95
   Thr Phe Asn Ile Ser Pro Leu Leu Ala Val Pro Trp Glu Ile Pro Thr
               100                 105                 110
   Tyr Ala Phe Ile Phe Thr Glu Arg Asn Pro Arg Thr Xaa
           115                 120                 125
<210> 9
   <211> 45
   <212> PRT
   <213> Homo sapiens
<400> 9
   Met Leu Asn Ala Thr Ser Ser Cys Glu Asp His Tyr Val Thr Ala Glu
   1               5                   10                  15
   Cys Asp Ile Cys Thr Cys Phe Leu His Tyr His Leu Ile Phe Thr Arg
               20                  25                  30
   Thr Leu Leu Cys Arg Phe Leu Met Thr Phe His Phe Ile
           35                  40                  45
<210> 10
   <211> 221
   <212> DNA
   <213> Homo sapiens
<400> 10
   acaggaagga cggtcagaga tgattccaac atgaggactt tgctagttgc agtgtctgac      60
   tcctcccatt ctcatagcag ttttgcacag tagttttggt ttgtgtgtat gttttaggca     120
   atgtgacaaa gcagttttcc tcctgcctcc ttgagaaatt taatcctgac aggttcttta     180
   agaaatcagc ttgatgtggg ctgattttaa aagaataaat g                         221
<210> 11
   <211> 621
   <212> DNA
   <213> Homo sapiens
<400> 11
   agggacgtat cactgtgtct tctcaaaatg gtgggtgtga atggaatttt tcaaagttga      60
   atcaatttga gatatttctg aagtttgttt aggaaacaag tatgtgacta ccttcctaag     120
   agctgactgg ttttttgagg cctggattgt gtgtgtatgt gcttatgtgt gtgttataaa     180
   atggaatcaa ctataccagg ttaaacaaat ggacaagtca tggaaaagac acagctctgt     240
   ttggacatta ataagggcgg gtgtggaagc tggaaagctg agatgccgga aggctcagtg     300
   cttagtaggc ccggaaaagc aatttttgaa ctggcatctc attccccttt tctaagacag     360
   gaaggacggt cagagatgat tccaacatga ggactttgct agttgcagtg tctgactcct     420
   cccattctca tagcagtttt gcacagtagt tttggtttgt gtgtatgttt taggcaatgt     480
   gacaaagcag ttttcctcct gcctccttga gaaatttaat cctgacaggt tctttaagaa     540
   atcagcttga tgtgggctga ttttaaaaga ataaatgaaa aaaaaaagct ggtgactgat     600
   agctgtttta ttggtcttgt a                                               621
<210> 12
   <211> 56
   <212> PRT
   <213> Homo sapiens
<400> 12
<210> 13
   <211> 356
   <212> DNA
   <213> Homo sapiens
<400> 13
   caagcacaaa gagcttgctc cctacgatga gaactggttc tacacgcgag ctgcttccac      60
   agcgcggcac ctgtacctcc ggggtggcgc tggggttggc tccatgacca agatctatgg     120
   gggacgtcag agaaacggcg tcatgcccag ccacttcagc cgaggctcca agagtgtggc     180
   ccgccgggtc ctccaagccc tggaggggct gaaaatggtg gaaaaggacc aagatggcgg     240
   ccgcaaactg acacctcagg gacaaagaga tctggacaga atcgccggac aggtggcagc     300
   tgccaacaag aagcattaga acaaaccatg ctgggttaat aaattgcctc attcgt        356
<210> 14
   <211> 873
   <212> DNA
   <213> Homo sapiens
<220>
   <221> CDS
   <222> (373)..(810)
   <223>
<400> 14
   gtactttcgc catcatagta ttctccacca ctgttccttc cagccacgaa cgacgcaaac      60
   gaagccaagt tcccccagct ccgaacagga gctctctatc ctctctctat tacactccgg     120
   gagaaggaaa cgcgggagga aacccaggcc tccacgcgcg accccttggc cctccccttt     180
   acctctccac ccctcactag acaccctccc ctctaggcgg ggacgaactt tcgccctgag     240
   agaggcggag cctcagcgtc taccctcgct ctcgcgagct ttcggaactc tcgcgagacc     300
   ctacgcccga cttgtgcgcc cgggaaaccc cgtcgttccc tttcccctgg ctggcagcgc     360
   ggaggccgca cg atg cct gga gtt act gta aaa gac gtg aac cag cag gag     411
   ttc gtc aga gct ctg gca gcc ttc ctc aaa aag tcc ggg aag ctg aaa       459
   gtc ccc gaa tgg gtg gat acc gtc aag ctg gcc aag cac aaa gag ctt       507
   gct ccc tac gat gag aac tgg ttc tac acg cga gct gct tcc aca gcg       555
   cgg cac ctg tac ctc cgg ggt ggc gct ggg gtt ggc tcc atg acc aag       603
   atc tat ggg gga cgt cag aga aac ggc gtc atg ccc agc cac ttc agc       651
   cga ggc tcc aag agt gtg gcc cgc cgg gtc ctc caa gcc ctg gag ggg       699
   ctg aaa atg gtg gaa aag gac caa gat ggc ggc cgc aaa ctg aca cct       747
   cag gga caa aga gat ctg gac aga atc gcc gga cag gtg gca gct gcc       795
   aac aag aag cat tag aacaaaccat gctgggttaa taaattgcct cattcgtaaa      850
   aaaaaaaaaa aaaaaaaaaa aaa                                             873
<210> 15
   <211> 145
   <212> PRT
   <213> Homo sapiens
<400> 15